# EUROPEAN PATENT SPECIFICATION

(11) **EP 0 806 414 B1**
(45) Date of publication and mention of the grant of the patent: **12.06.2002**
(21) Application number: 97112428.4
(22) Date of filing: 12.03.1991
(51) Int. Cl.: C07D 213/78, C07D 213/79, C07D 213/81, A01N 43/40

(54) **Intermediates for herbicidal carboxamide derivatives**
Zwischenprodukte für herbizide Carboxamidderivate
Intermédiaires pour des dérivés herbicides de carboxamide

(30) Priority: 16.03.1990 GB 9005965
(43) Date of publication of application: 12.11.1997
(62) Divisional of application: 91200546.9
(73) Proprietor: BASF AKTIENGESELLSCHAFT, 67056 Ludwigshafen (DE)
(72) Inventor: Foster, Christopher James, Faversham, Kent (GB); Gilkerson, Terence, Canterbury, Kent CT1 3NL (GB); Stocker, Richard, Rochester, Kent ME2 2PG (GB)

(56) References cited:
- EP-A- 0 053 789
- EP-A- 0 450 712
- EP-A- 0 488 474
- DE-A- 2 324 474
- US-A- 4 281 133
- CHEMICAL ABSTRACTS, vol. 79, no. 15, 15 October 1973 Columbus, Ohio, US; abstract no. 91940b, J. DELARGE ET AL.: "Synthesis and properties of some substituted anilinopyridines." page 415; XP002039809 & JOURNAL DE PHARMACIE DE BELGIQUE, vol. 28, no. 3, 1973, BRUSSELS, pages 283-290,

## Description

This invention relates to certain pyridinecarboxylic acid derivatives which are useful in the preparation of certain new herbicidal carboxamide derivatives. More specifically, the invention concerns novel phenoxypyridine carboxylic acid, acid halide, anhydride, mixed anhydride and ester compounds as well as certain halopyridine carboxamide compounds.

The herbicidal activity of N-phenyl-2-phenoxy-3-pyridine carboxamide compounds is known, for example from U.S. Patent Specifications Nos. 4270946 and 4327218, and from U.K. Patent Specification No. 2 087 887. One example in the U.K. Patent Specification No. 2 087 887 is the commercial compound N-(2,4-difluorophenyl)-2-(3-trifluoromethylphenoxy)-3-3-pyridine carboxamide (diflufenican).

This class of compounds, and diflufenican in particular, is discussed in detail in Pesticide Science 1987, 18, 15-28. In terms of the herbicidal utility of the compounds, emphasis is placed, in the Pesticide Science reference, upon their effectiveness in controlling broad-leaved weeds in winter cereals. The class of compounds is also described in "Synthesis and Chemistry of Agrochemicals", chapter 5, published by Am. Chem. Soc., (1987).

Certain N-phenyl-4-phenoxy-3-pyridine carboxamide compounds, and N-oxides thereof, are generally covered in Japanese Patent Application No. J6 3017-811A, for use with other active ingredients in herbicidal compositions. However in the examples only compounds having 4-phenoxy and 4-(3-chlorophenoxy) substituents are specifically disclosed (Compounds A31, A32 and A33).

There is no indication in the prior art references of any herbicidal activity in other structural isomers.

We have investigated other structural isomers of N-phenyl phenoxypyridine carboxamide compounds, and have found uniformly low or limited herbicidal activity in the following classes:
N-phenyl-2-phenoxy-4-pyridinecarboxamides,
N-phenyl-2-phenoxy-5-pyridinecarboxamides,
N-phenyl-3-phenoxy-5-pyridinecarboxamides,
N-phenyl-4-phenoxy-2-pyridinecarboxamides,
N-phenyl-3-phenoxy-2-pyridinecarboxamides,
N-phenyl-3-phenoxy-4-pyridinecarboxamides,
N-phenyl-3-phenoxy-6-pyridinecarboxamides.

However, we have surprisingly found excellent herbicidal activity in one particular class of compounds, N-phenyl-2-phenoxy-6-pyridinecarboxamides. Indeed comparison between the prior art commercial compound diflufenican and the closest compound in this new class of herbicidal compounds, the correspondingly substituted 2,6-isomer, shows a greater herbicidal activity against important representative grass species, Echinochloa crusgalli, and broadleaf species, Beta vulgaris, with an equally low action against the cereal species., Zea mays, for the 2,6-compound. A number of other compounds in this new group of herbicides exhibit yet greater activity than diflufenican against such representative grass and broadleaf species. These compounds form the subject of European Patent No 0447004 from which the present application has been divided.

The present application concerns novel pyridinecarboxylic acid derivatives which are useful in the preparation of the herbicidal carboxamide derivatives of EP-B-0447004.

EP-A-0434132 generically discloses certain optionally substituted 6-phenoxypyridinecarboxylic acid, 6-phenoxypyridinecarboxylic acid halide and 6-phenoxypyridinecarboxylic acid ester derivatives which are useful as intermediates in the preparation of certain herbicidal acrylonitrile derivatives. The point of attachment of the carboxylic acid, acid halide and ester groups to the pyridine moiety is unspecified in the generic formulae of these compounds. However, the specific examples disclose four 6-phenoxypyridinecarboxylic acid methyl ester derivatives in which the methyl ester group -CO-OCH₃ is attached at the 2-position of the pyridine moiety. No such acid halide or carboxylic acid derivatives having a 2,6-substitution pattern are disclosed.

EP-A-0053789 discloses certain 6-phenoxy-2-pyridinecarboxylic acid derivatives and the corresponding acid halides and mixed anhydrides which are useful as intermediates in the preparation of certain 2-pyridinecarboxamide derivatives which have anti-allergic activity. However, the phenoxy moiety of such compounds is unsubstituted.

US-A-4281133 discloses certain 6-phenoxy- and 6-phenylthio-2-pyridinecarboxylic acid methyl ester derivatives which are useful as intermediates in the preparation of certain substituted pyridine methyl esters of cyclopropane carboxylic acid which have insecticidal activity.

DE-A-2324474 discloses certain 6-phenoxypyridinecarboxylic acids and acid halides. However, the only compounds disclosed which have a 2,6-substitution pattern on the pyridine moiety have a phenoxy moiety which is either unsubstituted or substituted at the 4-position.

Chemical Abstracts, Vol. 79, No. 15, abstract no. 91940b (J. Delarge et al, J. Pharm.Belg., 1973, 28(3), pp. 283-90) discloses 6-chloro-N- (3-trifluoromethyl-phenyl)-2-pyridine carboxamide.

Accordingly, the present invention provides a compound of the general formula 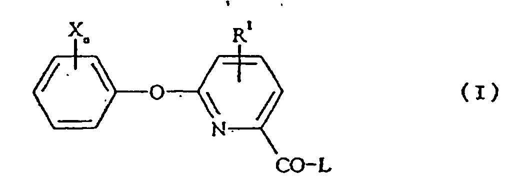 in which
R¹ represents a hydrogen or halogen atom or a C₁₋₆ alkyl or C₁₋₆ haloalkyl group; the or each group X independently represents a halogen atom, a C₁₋₆ alkyl or C₁₋₆ alkoxy group each optionally substituted by at least one substituent selected from halogen atoms, phenyl, cyano, amino, hydroxyl, C₁₋₆ alkoxy and (C₁₋₆ alkyl)amino groups or a C₂₋₆ alkenyloxy, C₂₋₆ alkynyloxy, cyano, carboxy, C₁₋₆ alkoxycarbonyl, (C₁₋₆ alkylthio)carbonyl, C₁₋₆ alkylcarbonyl, amido, C₁₋₆ alkylamido, nitro, C₁₋₆ alkylthio, C₁₋₆ haloalkylthio, C₂₋₆ alkenylthio, C₂₋₆ alkynylthio, C₁₋₆ alkylsulphinyl, C₁₋₆ alkylsulphonyl, C₁₋₆ alkyloximino-C₁₋₆ alkyl, or C₂₋₆ alkenyloximino-C₁₋₆ alkyl group;
n represents an integer from 1 to 5; one substituent X is located at the 3-position; and L is a leaving group selected from halogen atoms and hydroxy groups, and a group of the general formula -O-CO-Q, where Q represents an alkyl group or a group of the formula 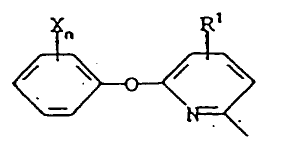 in which R¹, X and n are as defined above.

In general terms, unless otherwise stated herein, the term alkyl as used herein in respect of a radical or moiety refers to a straight or branched chain radical or moiety. Preferably, an alkyl moiety has from 1 to 4 carbon atoms. A preferred alkyl moiety is an ethyl or, especially, a methyl group and a preferred alkoxy moiety is ethoxy or, especially, methoxy.

Unless otherwise stated in this specification when an alkyl or alkoxy group is designated as being optionally substituted, the substituent groups which are optionally present are selected from halogen, especially fluorine, chlorine or bromine atoms, and phenyl, cyano, amino, hydroxy, alkoxy and (alkyl)amino groups, alkyl groups suitably having 1 or 2 carbon atoms. Preferred substituents are halogen, especially fluorine, atoms.

In general terms, unless otherwise stated, as used herein the term halogen atom may denote a bromine, iodine, chlorine or fluorine atom, and is especially a chlorine or fluorine atom.

An alkylamido group may have 1 or 2 alkyl groups, for example the group -CON(CH₃)₂.

The leaving group L may suitably be a bromine or, especially, a chlorine atom, or a group of the general formula -O-CO-Q as defined above. Preferably, L represents a chlorine atom.

Compounds of general formula I in which R¹ represents a hydrogen atom are preferred and compounds of formula 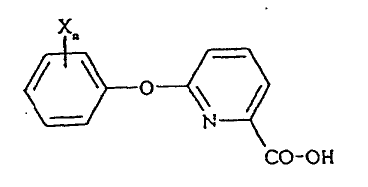 in which X and n are as defined above are particularly preferred.

Suitably, the or each group X independently represents a halogen atom, especially a bromine, chlorine or fluorine atom, an alkoxy group, especially methoxy, an alkyl group, especially methyl or ethyl, a haloalkyl group, preferably a fluoroalkyl and/or a halomethyl group, especially a trifluoromethyl group, a nitro group, a carboxy group, an alkoxycarbonyl group, especially methoxycarbonyl, an alkylthiocarbonyl group, especially ethylthiocarbonyl, an amido group, an alkylamido group, especially dimethylamido, an alkylthio, group, especially methylthio, an alkylsulphonyl group, especially methylsulphonyl, an alkyloximinoalkyl group, for example (1-ethoxyiminoethyl), a haloalkoxy group, preferably a fluoroalkoxy and/or a halomethoxy group, preferably a trifluoromethoxy group, an alkenyloxy group, for example allyloxy group, or a cyano group.

Preferably, n is 1 or 2 and the or each group X independently represents a halogen atom, an alkyl group, an alkoxy group, a haloalkyl group, a haloalkoxy group, a nitro group, an alkenyloxy group, an alkylthio group, an alkylsulphonyl group, an alkyl-oximinoalkyl group or a cyano group.

One substituent X is located at the 3-position, and is preferably a fluorine, chlorine or bromine atom or a trifluoromethyl, cyano, trifluoromethoxy or ethyl group.

Most preferably, Xₙ represents a trifluoromethyl or 3-cyano substituent.

The invention also provides compounds of formula 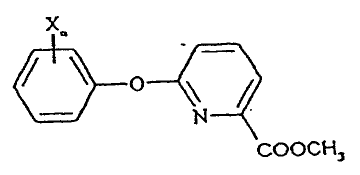 in which Xₙ is selected from the group consisting of 2,4-diCl and 3,5-diCl.

Preferably, the compounds of fomula I are obtainable from the corresponding phenoxy-substituted picolinic acid by reaction with thionyl chloride.

The invention also provides a process for preparing a compound of formula I in which R¹, X and n are as defined above and L is a chlorine atom which comprises reacting a compound of formula I in which R¹, X and n are as defined above and L is a hydroxy group with thionyl chloride.

In another aspect, the invention provides a compound of the general formula 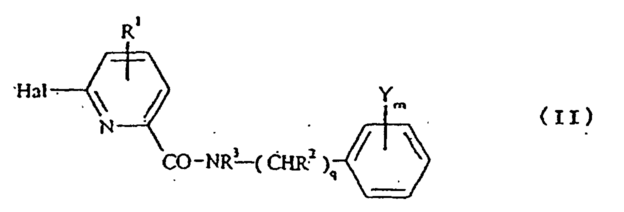 in which R¹ represents a hydrogen or halogen or a C₁₋₆ alkyl or C₁₋₆ haloalkyl group, preferably a hydrogen atom,
Hal represents a halogen atom;
R² represents a hydrogen atom or a C₁₋₆ alkyl group;
q represents 0 or 1;
R³ represents a hydrogen atom or a C₁₋₆ alkyl or C₂₋₆ alkenyl group;
the or each group Y independently represents a halogen atom or a C₁₋₆ alkyl, nitro, cyano, C₁₋₆ lialoalkyl, C₁₋₆ alkoxy or C₁₋₆ haloalkoxy group;
m represents an integer from 1 to 5; and
one substituent Y is located at the 4-position of the phenyl radical.
Suitably, Hal represents a bromine or, especially, a chlorine atom.

Preferably R² represents a hydrogen atom or a methyl group, so that, when q is 1, -(CHR²)_{q}-represents a methylene or ethylidene group. However, q is especially 0.

Preferably R³ represents a methyl, ethyl or allyl group or a hydrogen atom. Most preferably, R³ represents a hydrogen atom or a methyl group.

Suitably, the or each group Y independently represents a halogen atom, especially an iodine, chlorine or fluorine atom, a C₁₋₄ alkyl group especially methyl, a nitro or cyano group, a C₁₋₄ haloalkyl group, especially trifluoromethyl, a C₁₋₄ alkoxy group, especially methoxy, or a C₁₋₄ haloalkoxy group, especially trifluoromethoxy.

m preferably represents 1, 2 or 3, most preferably 1 or 2.

Preferably, m is 1 and the group Y represents a halogen atom, or a cyano, methyl or trifluoromethyl group, or m is 2 or 3 and at least one Y is a halogen atom.

When m is at least 2, there are suitably substituents Y at the 2- and 4-positions. When m is at least 2, the substituents Y may be different but are preferably identical.

In certain preferred compounds Yₘ represents 4-fluoro.

In certain preferred compounds Yₘ represents 2,4-difluoro.

In certain preferred compounds Yₘ represents 4-methyl.

In certain preferred compounds Yₘ represents 4-trifluoromethyl.

In certain preferred compounds Yₘ represents 2,4,5-trifluoro or 2,3,4-trifluoro.

In certain preferred compounds Yₘ represents 4-chloro.

In certain preferred compounds Yₘ represents 4-cyano.

It will be appreciated that certain of the compounds of the invention, for example those in which q is 1 and R² is other than hydrogen, will exist in different stereoisomeric forms. The present invention is to be understood to include all individual stereoisomeric forms of the compounds of general formula I and mixtures thereof in whatever proportion. It will be further appreciated that one stereoisomer may have a greater activity than another stereoisomer of the same compound or than a mixture of the isomers.

Compounds of the general formula I, methyl-6-(2,4-dichlorophenoxy)picolinate and methyl-6-(3,5-dichlorophenoxy)picolinate may be prepared from corresponding phenoxy-substituted picolinic acids by standard methods for the preparation of, for example, esters using, for example, alcohols and acid catalysts or thionyl chloride, or of acid chlorides, bromides, anhydrides or mixed anhydrides, using, for example, thionyl chloride, thionyl bromide or acetic anhydride. The acid compounds themselves can be prepared by standard methods from chloropicolinic acid or ester thereof. Chloropicolinic acid, or ester thereof, may be prepared by the method described in J. Pharm. Belg. (1980), 35 1, 5-11.

Compounds of the general formula II may suitably be prepared by reacting an appropriately substituted aniline or amine with a 2-halo-6-pyridine carboxylic acid derivative of the general formula 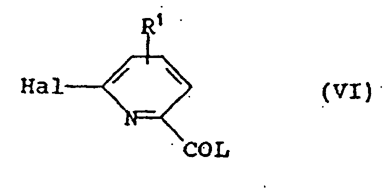 in which R¹, Hal and L are as defined above. Compounds of formula VI may be prepared by conventional techniques from picolinic acid.

The compounds of the present invention may be isolated and purified by conventional techniques, for example by solvent extraction, evaporation followed by recrystallisation or by chromatography on silica.

The following Examples illustrate the invention. Examples 1 to 17 relate to the preparation of various classes of starting materials, compounds of general formula I, methyl-6-(2,4-dichlorophenoxy)picolinate and methyl-6-(3,5-dichlorophenoxy)picolinate and Examples 18 and 19 to the preparation of compounds of general formula II. All structures were confirmed by mass spectroscopy and 300'H nmr.

### Example 1

### Preparation of methyl-6-(3-α,α,α-trifluoromethylphenoxy)picolinate

A solution of sodium methoxide (from 1.3g sodium in 20 ml methanol) was added to a solution of 3-α,α,α-trifluoromethylphenol (8.9g) in xylene (50ml). The solvents were evaporated in vacuo to give the dry sodium phenolate. Pyridine (25ml) and xylene (50ml) were added, followed by cuprous chloride (1.5g) and the mixture heated to reflux. A solution of methyl-6-chloropicolinate (8.5g) in xylene (50ml) was added dropwise. The mixture was refluxed for a further 14 hours. After cooling, the mixture was poured into water (500ml) and acidified with dilute sulphuric acid. The xylene layer was separated and the aqueous layer further extracted with diethyl ether. The combined extracts were washed with brine, dried over anhydrous magnesium sulphate and evaporated. The residue was purified on a silica gel column using 5% (v/v) diethyl ether dichloromethane as eluant to give the title compound (7.4g) as a yellow solid of melting point 43-44°C. m/e Theory : Found 297 : 297.

| | | | |
|---|---|---|---|
| Theory for C₁₄H₁₀O₃NF₃ | C 56.6 | H 3.4 | N 4.7% |
| Found | C 55.6 | H 3.4 | N 4.8% |

The compounds of general formula X listed in Table I below were prepared by the method of Example 1.

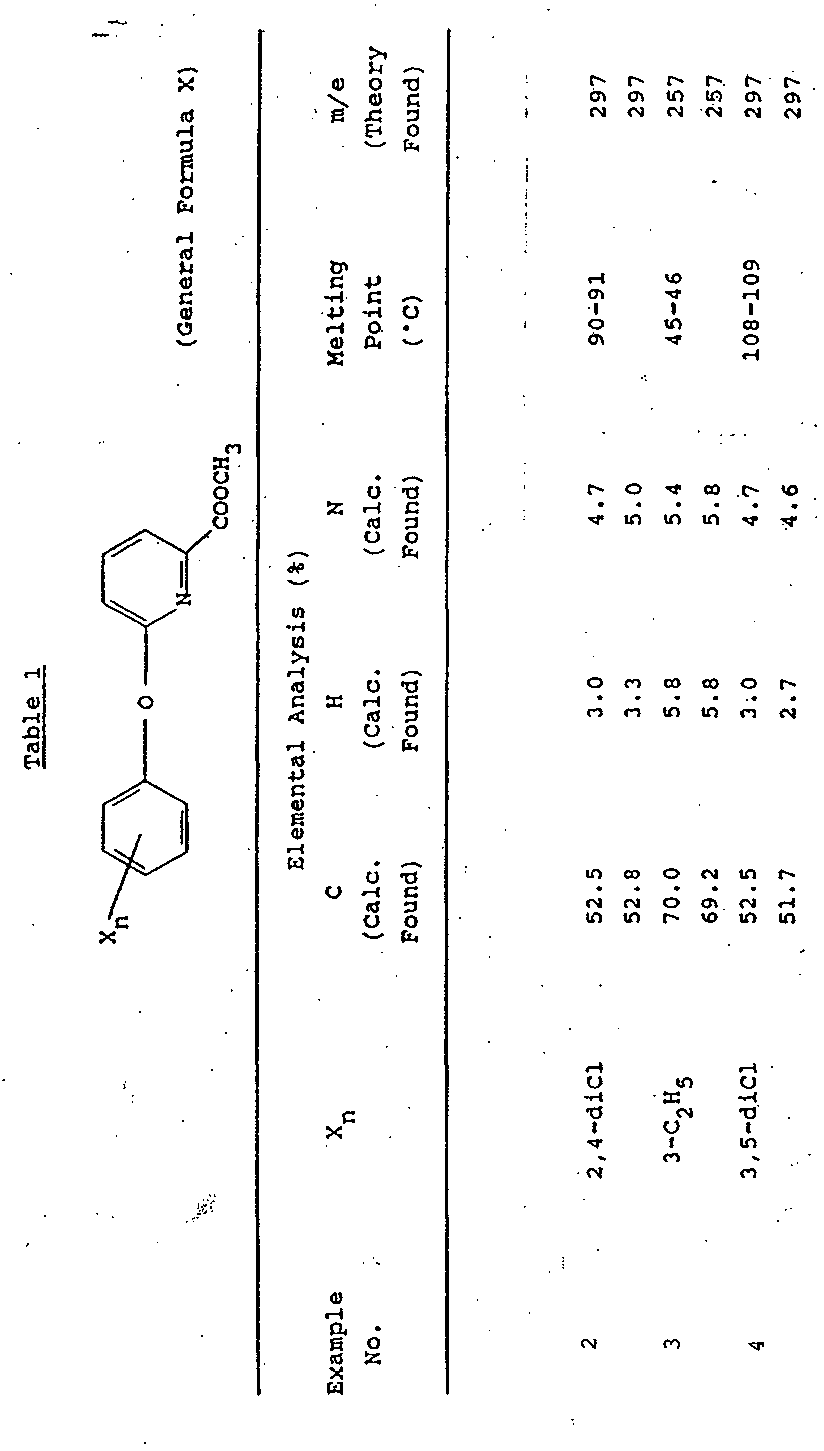

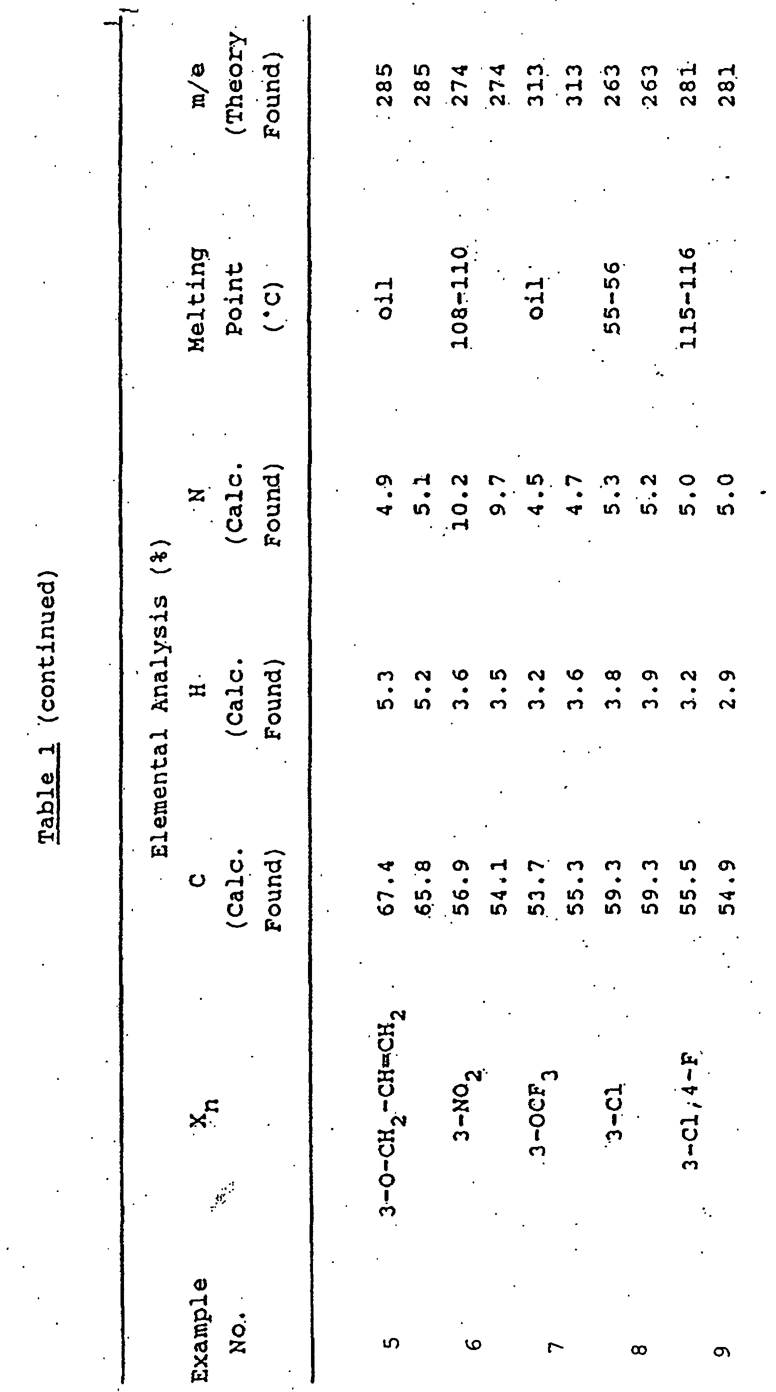

### Example 10

### Preparation of 6-(3-α,α,α-trifluoromethylphenoxy) picolinic acid

A solution of methyl-6-(3-α,α,α-trifluoromethylphenoxy)-picolinate (8g) in 50% aqueous methanol (45 ml) containing sodium hydroxide (2.5g) was refluxed for 3 hours. The methanol was evaporated, more water added to the residue and the aqueous solution extracted with diethyl ether. The aqueous phase was then acidified with dilute hydrochloric acid to precipitate the title compound (7.1g) as a white solid of melting point 92-93°C. m/e Theory : Found 283:283

| | | | |
|---|---|---|---|
| Theory for C₁₃H₈NO₃F₃ | C 55.1 | H 2.8 | N 4.9% |
| Found | C 53.3 | H 3.0 | N 5.0% |

The compounds of general formula XX listed in Table 2 below were prepared by the method of Example 10.

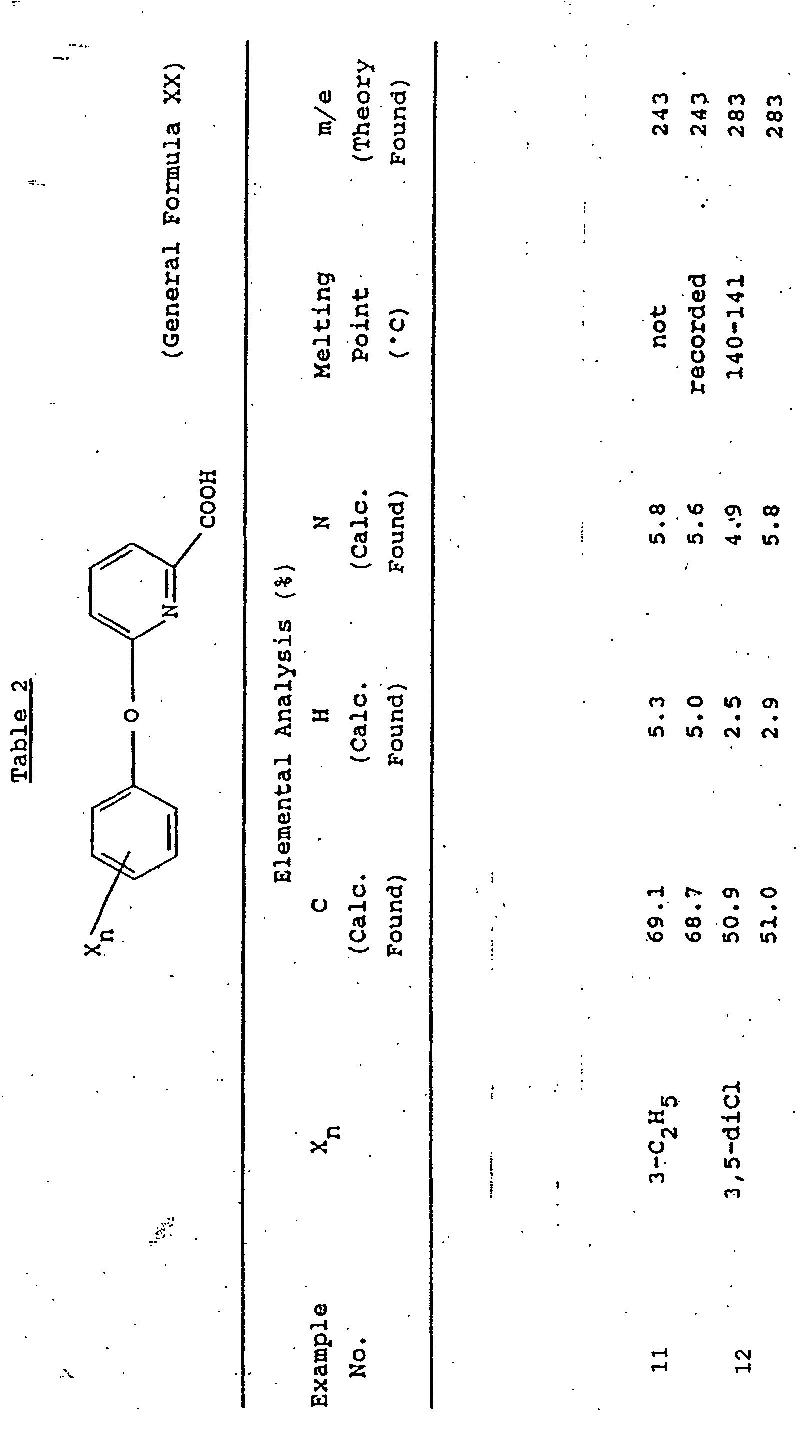

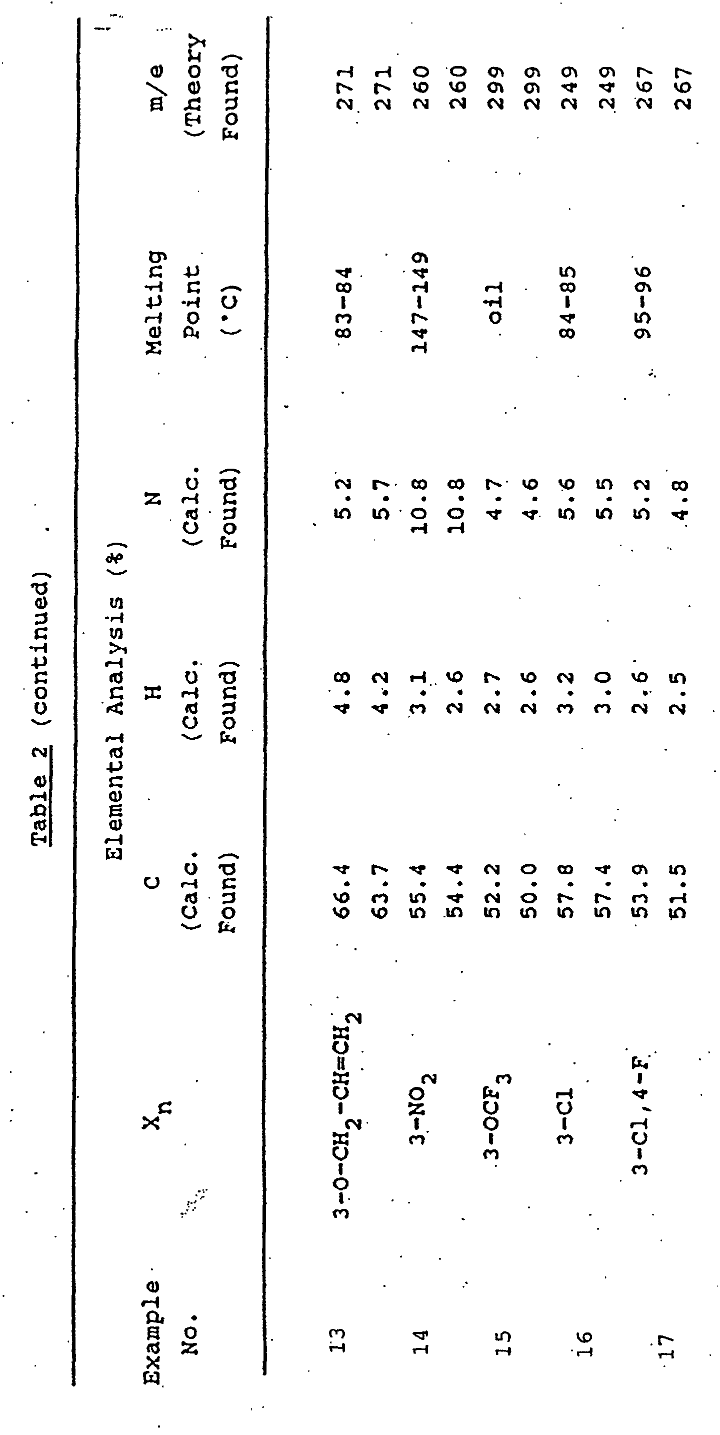

### Example 18

### Preparation of N-(2,4-difluorophenyl)-2-chloro-6-pyridinecarboxamide

6-Chloropicolinic acid (25g) in thionyl chloride (50ml) was stirred and heated to reflux for 1.5 hours. The excess thionyl chloride was evaporated in vacuo and dichloromethane (100ml) added to the residual 6-chloropicolinoyl chloride. A solution of 2,4-difluoroaniline (20.5g) and triethylamine (16g) in dichloromethane (50ml) was added with stirring, maintaining the temperature below 20°C. After the addition, the reaction mixture was stirred a further 1 hour at ambient temperature. Water was then added to the reaction mixture and the dichloromethane phase separated. After a further washing with water, brine and drying over anhydrous magnesium sulphate, the dichloromethane was removed to give the title compound (42g) as a pale yellow solid of melting point 86-87°C.
m/e Theory : Found 268:268

| | | | |
|---|---|---|---|
| Theory for C₁₂H₇N₂OF₂Cl | C 53.7 | H 2.6 | N 10.4% |
| Found | C 53.6 | H 2.7 | N 10.5% |

The compounds of general formula II listed in Table 3 below were prepared by the method of Example 18.

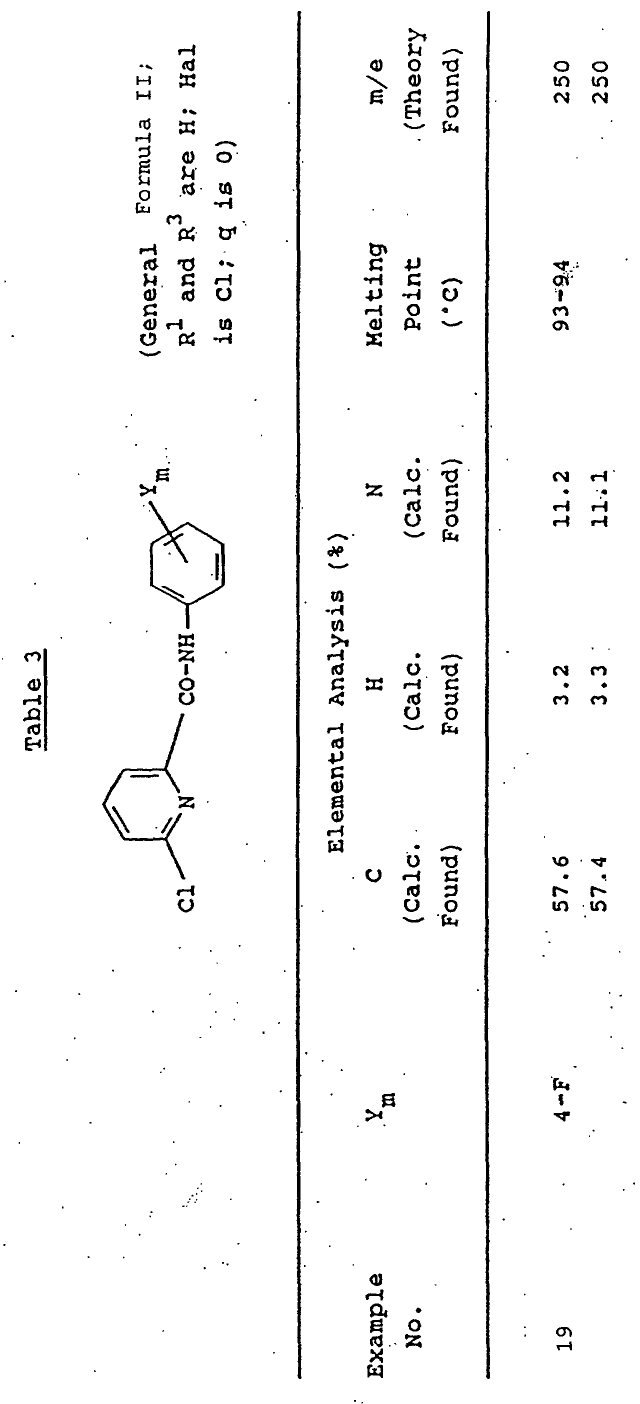

## Claims

1. A compound of the general formula 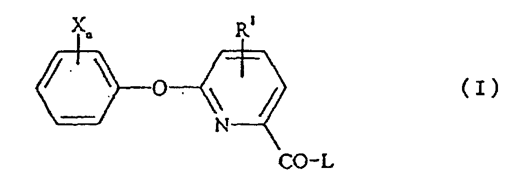 in which
R¹ represents a hydrogen or halogen atom or a C₁₋₆ alkyl or C₁₋₆ haloalkyl group; the or each group X independently represents a halogen atom, a C₁₋₆ alkyl or C₁₋₆ alkoxy group each optionally substituted by at least one substituent selected from halogen atoms, phenyl, cyano, amino, hydroxyl, C₁₋₆ alkoxy and (C₁₋₆ alkyl)amino groups or a C₂₋₆ alkenyloxy, C₂₋₆ alkynyloxy, cyano, carboxy, C₁₋₆ alkoxycarbonyl, (C₁₋₆ alkylthio)carbonyl, C₁₋₆ alkylcarbonyl, amido, C₁₋₆ alkylamido, nitro, C₁₋₆ alkylthio, C₁₋₆ haloalkylthio, C₂₋₆ alkenylthio, C₂₋₆ alkynylthio, C₁₋₆ alkylsulphinyl, C₁₋₆ alkylsulphonyl, C₁₋₆ alkyloximino-C₁₋₆ alkyl, or C₂₋₆ alkenyloximino-C₁₋₆ alkyl group;
n represents an integer from 1 to 5; one substituent X is located at the 3-position; and
L is a leaving group selected from halogen atoms and hydroxy groups, and a group of the general formula -O-CO-Q, where Q represents an alkyl group or a group of the formula 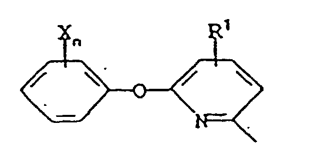 in which R¹, X and n are as defined above.

2. A compound according to claim 1 wherein R¹ represents a hydrogen atom.

3. A compound of formula 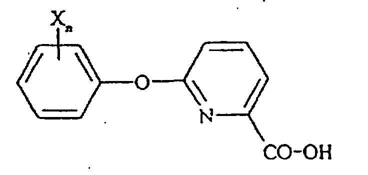 in which X and n are as defined in claim 1.

4. A compound as claimed in any one of the preceding claims, wherein Xₙ represents a 3-trifluoromethyl or 3-cyano group

5. A compound of formula 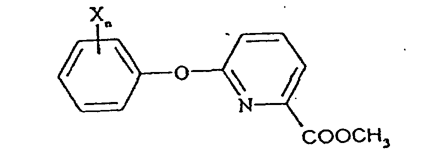 in which Xₙ is selected from the group consisting of 2,4-diCl. and 3,5-diCl.

6. A compound as claimed in claim 1 obtainable from the corresponding phenoxy-substituted picolinic acid by reaction with thionyl chloride.

7. A process for the preparation of a compound as defined in claim 1 in which R¹,X and n are as defined in claim 1 and L is a chlorine atom which comprises reacting a compound as defined in claim 1 in which R¹,X and n are as defined in claim 1 and L is a hydroxy group with thionyl chloride.

8. A compound of the general formula 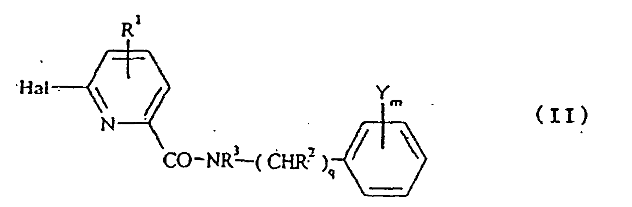 in which
R¹ is as defined in claim 1;
Hal represents a halogen atom;
R² represents a hydrogen atom or a C₁₋₆ alkyl group;
q represents 0 or 1;
R³ represents a hydrogen atom or a C₁₋₆ alkyl or C₂₋₆ alkenyl group;
the or each group Y independently represents a halogen atom or a C₁₋₆ alkyl, nitro, cyano, C₁₋₆ haloalkyl, C₁₋₆ alkoxy or C₁₋₆ haloalkoxy group;
m represents an integer from 1 to 5; and
one substituent Y is located at the 4-position of the phenyl radical.

9. A compound according to claim 8 wherein R¹ represents a hydrogen atom.

## Patentansprüche

1. Verbindung der allgemeinen Formel 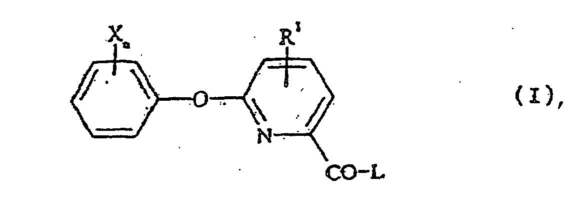 in der
R¹ ein Wasserstoff- oder Halogenatom oder eine C₁₋₆Alkyloder C₁₋₆Halogenalkylgruppe darstellt;
die Gruppe X bzw. jede Gruppe X unabhängig ein Halogenatom oder eine C₁₋₆Alkyl- oder C₁₋₆Alkoxygruppe darstellt, die jeweils gegebenenfalls durch mindestens einen Substituenten aus der Reihe Halogenatome, Phenylgruppe, Cyangruppe, Aminogruppe, Hydroxygruppe, C₁₋₆Alkoxygruppe und (C₁₋₆Alkyl)aminogruppe substituiert ist, oder eine C₂₋₆Alkenyloxy-, C₂₋₆Alkinyloxy-, Cyan-, Carboxy-, C₁₋₆Alkoxycarbonyl-, (C₁₋₆Alkylthio)carbonyl-, C₁₋₆Alkylcarbonyl-, Amido-, C₁₋₆Alkylamido-, Nitro-, C₁₋₆Alkylthio-, C₁₋₆Halogenalkylthio-, C₂₋₆Alkenylthio-, C₂₋₆Alkinylthio-, C₁₋₆Alkylsulfinyl-, C₁₋₆Alkylsulfonyl-, C₁₋₆Alkyloximino-C₁₋₆alkyl- oder C₂₋₆Alkenyloximino-C₁₋₆alkylgruppe bedeutet;
n eine ganze Zahl von 1 bis 5 bedeutet; ein Substituent X in 3-Stellung steht; und
L eine Abgangsgruppe aus der Reihe Halogenatome und Hydroxygruppen bedeutet, sowie eine Gruppe der allgemeinen Formel -O-CO-Q, in der Q eine Alkylgruppe oder eine Gruppe der Formel 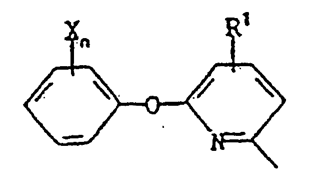 in der R¹, X und n wie oben definiert sind, darstellt, bedeutet.

2. Verbindung nach Anspruch 1, wobei R¹ ein Wasserstoffatom bedeutet.

3. Verbindung der Formel 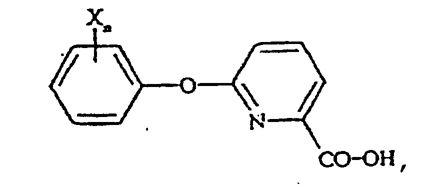 in der X und n wie in Anspruch 1 definiert sind.

4. Verbindung nach einem der vorhergehenden Ansprüche, wobei Xₙ eine 3-Trifluormethyl- oder 3-Cyangruppe bedeutet.

5. Verbindung der Formel 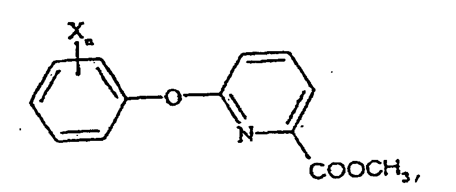 in der Xₙ aus der Gruppe 2,4-DiCl und 3,5-DiCl stammt.

6. Verbindung nach Anspruch 1, erhältlich durch Umsetzen der entsprechenden Phenoxy-substituierten Picolinsäure mit Thionylchlorid.

7. Verfahren zur Herstellung einer wie in Anspruch 1 definierten Verbindung, in der R¹, X und n wie in Anspruch 1 definiert sind und L ein Chloratom bedeutet, bei dem man eine wie in Anspruch 1 definierte Verbindung, in der R¹, X und n wie in Anspruch 1 definiert sind und L eine Hydroxygruppe bedeutet, mit Thionylchlorid umsetzt.

8. Verbindung der allgemeinen Formel 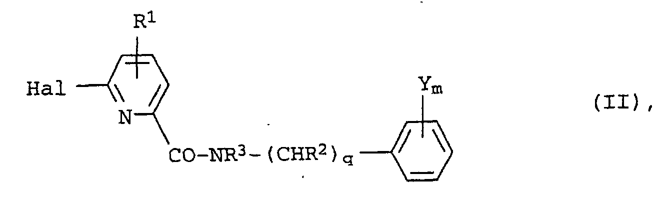 in der
R¹ wie in Anspruch 1 definiert ist;
Hal ein Halogenatom bedeutet;
R² ein Wasserstoffatom oder eine C₁₋₆Alkylgruppe bedeutet;
q 0 oder 1 bedeutet;
R³ ein Wasserstoffatom oder eine C₁₋₆Alkyl- oder C₂₋₆Alkenylgruppe bedeutet;
die Gruppe Y bzw. jede Gruppe Y unabhängig ein Halogenatom oder eine C₁₋₆Alkyl-, Nitro-, Cyan-, C₁₋₆Halogenalkyl-, C₁₋₆Alkoxy- oder C₁₋₆Halogenalkoxygruppe bedeutet;
m eine ganze Zahl von 1 bis 5 bedeutet; und
ein Substituent Y in 4-Stellung des Phenylrests steht.

9. Verbindung nach Anspruch 8, wobei R¹ ein Wasserstoffatom darstellt.

## Revendications

1. Composé de formule générale 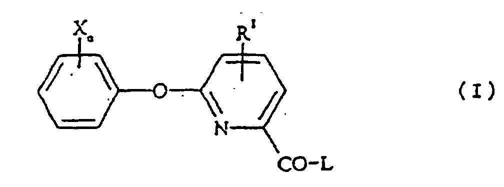 dans laquelle
R¹ représente un atome d'hydrogène ou d'halogène ou un groupe alkyle en C₁-C₆ ou halogénoalkyle en C₁-C₆ ;
le ou chaque groupe X représente indépendamment un atome d'halogène, un groupe alkyle en C₁-C₆ ou un groupe alcoxy en C₁-C₆, chacun étant facultativement substitué par au moins un substituant choisi parmi les atomes d'halogène et les groupes phényle, cyano, amino, hydroxyle, alcoxy en C₁-C₆ et (alkyle en C₁-C₆)amino, ou un groupe alcényloxy en C₂-C₆, alcynyloxy en C₂-C₆, cyano, carboxy, (alcoxy en C₁-C₆)carbonyle, (alkylthio en C₁-C₆)-carbonyle, (alkyle en C₁-C₆)carbonyle, amido, (alkyle en C₁-C₆)amido, nitro, alkylthio en C₁-C₆, halogénoalkylthio en C₁-C₆, alcénylthio en C₂-C₆, alcynylthio en C₂-C₆, alkylsulfinyle en C₁-C₆, alkylsulfonyle en C₁-C₆, alkyloximino en C₁-C₆-alkyle en C₁-C₆ ou alcényloximino en C₂-C₆-alkyle en C₁-C₆ ;
n représente un nombre entier de 1 à 5 ; un substituant X est situé à la position 3 ; et
L est un groupe partant choisi parmi les atomes d'halogène et les groupes hydroxyle, et un groupe de formule générale -O-CO-Q où Q représente un groupe alkyle ou un groupe de formule 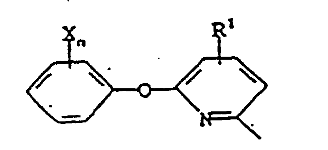 où R¹, X et n sont tels que définis ci-dessus.

2. Composé selon la revendication 1, dans lequel R¹ représente un atome d'hydrogène.

3. Composé de formule 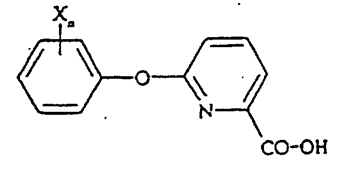 dans laquelle X et n sont tels que définis dans la revendication 1.

4. Composé selon l'une quelconque des revendications précédentes, dans lequel Xₙ représente un groupe 3-trifluorométhyle ou 3-cyano.

5. Composé de formule 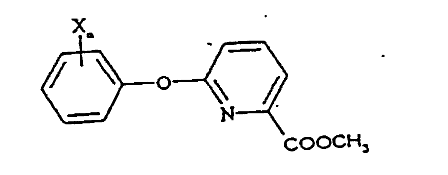 dans laquelle Xₙ est choisi dans la classe formée par 2,4-diCl et 3,5-diCl.

6. Composé selon la revendication 1, pouvant être obtenu à partir de l'acide picolinique à substitution phénoxy correspondant par réaction avec le chlorure de thionyle.

7. Procédé pour la préparation d'un composé tel que défini dans la revendication 1 dans lequel R¹, X et n sont tels que définis dans la revendication 1 et L est un atome de chlore, qui comprend la réaction d'un composé tel que défini dans la revendication 1 dans lequel R¹, X et n sont tels que définis dans la revendication 1 et L est un groupe hydroxyle avec le chlorure de thionyle.

8. Composé de formule générale 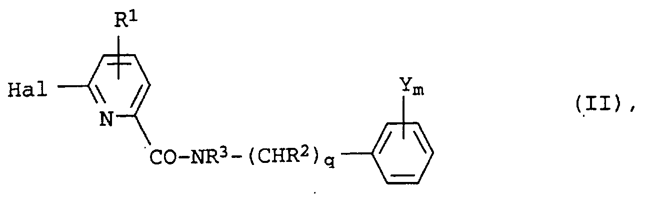 dans laquelle
R¹ est tel que défini dans la revendication 1 ;
Hal représente un atome d'halogène ;
R² représente un atome d'hydrogène ou un groupe alkyle en C₁-C₆ ;
q représente 0 ou 1 ;
R³ représente un atome d'hydrogène ou un groupe alkyle en C₁-C₆ ou alcényle en C₂-C₆ ;
le ou chaque groupe Y représente indépendamment un atome d'halogène ou un groupe alkyle en C₁-C₆, nitro, cyano, halogénoalkyle en C₁-C₆, alcoxy en C₁-C₆ ou halogénoalcoxy en C₁-C₆ ;
m représente un nombre entier de 1 à 5 ; et
un substituant Y est situé à la position 4 du radical phényle.

9. Composé selon la revendication 8, dans lequel R¹ représente un atome d'hydrogène.
